# EUROPEAN PATENT SPECIFICATION

(11) **EP 2 839 829 B1**
(45) Date of publication and mention of the grant of the patent: **26.09.2018**
(21) Application number: 13778634.9
(22) Date of filing: 17.04.2013
(51) Int. Cl.: A61K 9/22, A61K 9/20, A61K 47/30, A61K 31/495, A61K 9/24, A61K 9/28

(54) **SUSTAINED RELEASE TABLET CONTAINING LEVODROPROPIZINE AND METHOD FOR PREPARING SAME**
TABLETTE MIT VERZÖGERTER FREISETZUNG ENTHALTEND LEVODROPROPIZIN UND VERFAHREN ZUR HERSTELLUNG DAVON
COMPRIMÉ À LIBÉRATION PROLONGÉE CONTENANT DE LA LÉVODROPROPIZINE ET PROCÉDÉ POUR PRÉPARER CELUI-CI

(30) Priority: 17.04.2012 KR 20120039907
(43) Date of publication of application: 25.02.2015
(73) Proprietor: Korea United Pharm. Inc., Jeondong-myeon, Yeongi-gun, Chungcheongnam-do 339-840 (KR)
(72) Inventor: CHOI, Youn-Woong, Ansan-si Gyeonggi-do 425-726 (KR); CHO, Sang-Min, Suwon-si, Gyeonggi-do 443-270 (KR); MIN, Byung-Gu, Seoul 122-040 (KR); KIM, Bo-Kyung, Seoul 151-901 (KR); JANG, Jae-Sang, Incheon 406-840 (KR); KANG, Hyun-Ju, Anyang-si Gyeonggi-do 431-060 (KR)
(74) Representative: Vossius & Partner Patentanwälte Rechtsanwälte mbB
(86) International application number: PCT/KR2013/003232
(87) International publication number: WO 2013/157841

(56) References cited:
- WO-A2-2011/126327
- KR-A- 20100 089 532
- KR-A- 20110 113 413
- KR-A- 20110 132 170
- KR-A- 20120 033 557
- KR-A- 20120 033 557

## Description

### Technical Field

The present invention relates to a specific levodropropizine-containing sustained release tablet as defined in claim 1.

More particularly, the present invention relates to a levodropropizine-containing sustained release tablet for retarding drug absorption by controlling drug release through a drug design where levodropropizine is in mixture with a polymer compound.

### Background Art

Levodropropizine inhibits impulse transmission via group C nerve fibers, a class of peripheral nerves involved in the cough reflex, thus exhibiting effectiveness in suppressing symptoms caused by neuropeptides at the nerve endings of group C nerve fibers, such as inflammatory reactions, bronchospasm, airway hypersensitivity, mucus hypersecretion, vascular permeability, and triggering of the cough reflex.

When administered orally, levodropropizine is known to be absorbed at a rate of at least 75 %, with a peak serum level being attained at about 0.25 to 1 hour after oral administration. Then, approximately 35 % of the administered levodropropizine is excreted, with a half life of about 1 hr in the body.

Currently, levodropropizine is commercially available in a tablet form, with the regimen of one 60 mg tablet per oral administration, three times a day.

Levodropropizine formulations in current use exert rapid pharmaceutical effects because they are dissolved and absorbed as soon as they have been administered. However, there is an increase of necessity for a sustained release formulation of levodropropizine that is designed to maintain an effective serum level of the drug for a prolonged period of time, thereby being expected to provide the advantage of alleviating the fluctuation of serum levels caused by frequent administrations, with the concomitant reduction of the side effects accompanying the fluctuating serum levels, and decreasing the frequency of administration, i.e., three times a day, to improve drug compliance.

As a related art, Korean Patent Application Unexamined Publication No. 10-2011-0113413 discloses a sustained-release pharmaceutical composition containing levodropropizine, and a preparation method thereof.

WO 2011/126327 A2 relates to a pharmaceutical composition with controlled-release properties comprising mosapride or levodropropizine. KR 2012 0033557 A describes a pharmaceutical composition comprising a specific immediate release part as well as a specific sustained release part.

In achieving the sustained release of levodropropizine, it is important to provide not only a high dissolution rate in the early stages of administration, but also to maintain a constant effective serum level for up to 12 hours after administration for dosage forms designed to meet a requirement for the rapid exertion and maintenance of pharmaceutical effects, such as bilayer tablets, double-layer tablets, and multi-layer tablets (comprising an immediate release layer and a sustained release layer).

### Disclosure

### Technical Problem

Leading to the present invention, thorough and intensive research into a levodropropizine-containing, sustained release tablet resulted in the finding that when a matrix-type sustained release portion containing highly viscous hydroxypropylmethylcellulose-surrounded levodropropizine is formulated with an immediate release portion, responsible for rapid therapy at an early stage, containing a single tablet dosage of levodropropizine, the resulting formulation can rapidly exert and maintain the pharmaceutical effect of levodropropizine.

It is therefore an object of the present invention to provide a levodropropizine-containing, sustained release tablet that has a multilayer structure composed of an immediate-release layer and a sustained release layer, each containing levodropropizine as an active ingredient, and which thus can be administered twice a day based on a dose of 60 mg; in contrast to conventional tablets administered three times a day, and which has the advantages of reaching a therapeutically effective serum level at an early stage and maintaining the effective serum level for a prolonged duration, which leads to a synergic effect of therapy, thereby improving patients' convenience and drug compliance due to the simplified regimen.

### Technical Solution

In accordance with an aspect thereof, the present invention provides a sustained release tablet of levodropropizine as defined in claim 1, comprising an immediate-release layer containing levodropropizine and a sustained-release layer containing levodropropizine and a release-controlling polymer.

### Advantageous Effects

In addition to exhibiting antitussive expectoration immediately upon oral administration, the bilayered sustained release tablet of levodropropizin comprising an immediate-release layer and a sustained-release layer can maintain a certain serum level of levodropropizine for a sustained period of time and thus can be administered at a reduced frequency, which in turn leads to an improvement in the drug adaptability and drug compliance of patients.

Hence, the sustained release tablet of levodropropizine according to the present invention overcomes the problem of three dosages per day and is advantageous in administration and therapeutic efficacy.

### Description of Drawings

FIG. 1 shows dissolution profiles of levodropropizine from immediate-release formulations.
FIG. 2 shows dissolution profiles of levodropropizine from sustained-release formulations.
FIG. 3 shows dissolution profiles of levodropropizine from the sustained-release tablet of the present invention.
FIGS. 4 and 5 shows dissolution profiles of levodropropizine from the sustained-release tablet of the present invention in comparison with those of a control tablet.
FIG. 6 shows serum levels of levodropropizine after oral administration of 'dropizine' tablet and 'UI04LDP090CT' having the composition of Example 3 to beagle dogs

### Best Mode

Below, a detailed description will be given of the present invention

The present invention addresses a sustained release tablet of levodropropizine as defined in claim 1, comprising an immediate release layer containing levodropropizine and a sustained release layer containing levodropropizine and a release-controlling polymer.

Herein disclosed is a sustained -release tablet of levodropropizine, wherein the content of levodropropizine is in the order of 10 to 70 mg in the immediate release layer and in the order of 20 to 80 mg in the sustained layer. More particularly, levodropropizine is contained in an amount of 30 to 60 mg in each of the immediate release layer and the sustained release layer.

The sustained release layer within the sustained release tablet of levodropropizine
contains a release-controlling polymer. If it is pharmaceutically acceptable, any release-controlling polymer may be employed. The polymer may be selected from the group consisting of a cellulose derivative, such as: hydroxypropyl methyl cellulose, methyl cellulose, ethyl cellulose, hydroxypropyl cellulose, and sodium carboxymethyl cellulose; propylene oxide and derivatives thereof; polyvinylpyrrolidone (Mw. 90, brand name Povidone K-90); polyethylene glycol, polyvinyl alcohols; polyvinylacetate, polyvinylacetate phthalate; polymethacrylate, polymers of polymethacrylate (commercially available as Eudragit), polyacrylic acid, polymethacrylate derivatives (Carbomer, etc.); glycerolmonostearate; Poloxamer; and a combination thereof.

Preferable is at least one selected from the group consisting of: hydroxypropylmethylcellulose, carbomer, hydroxypropylcellulose, methylcellulose, polyvinylpyrrolidone, and polyvinylalcohol.

Herein disclosed is a sustained -release tablet of levodropropizine, wherein the sustained release tablet of levodropropizine contains the release-controlling polymer in an amount of 10 to 80 % by weight based on the total weight thereof. More preferably, the content of the release-controlling polymer is in the order of 10 to 60 % by weight.

Preferably, the release-controlling polymer has a viscosity of 60,000 to 140,000 cps.

For hydroxypropylmethylcellulose, commercially available products with various viscosities are known. The dissolution rate of the sustained tablet of levodropropizine can be adjusted by adjusting the viscosity of hydroxypropylmethylcellulose.

The hydroxypropylmethylcellulose may be of high viscosity. Preferably, it has a viscosity of 60,000 cps to 140,000 cps. For example, a viscosity less than 60,000 cps requires a great amount of hydroxypropylmethylcellulose, which leads to an increase in the size of the tablet. On the other hand, hydroxypropylmethylcellulose with a viscosity higher than 140,000 cps is difficult to homogeneously mix with the drug.

Each of the immediate-release layer and the sustained-release layer in the sustained-release tablet of levodropropizine as defined in claim 1 may further comprise a pharmaceutically acceptable excipient in addition to levodropropizine or a combination of levodropropizine and the release-controlling polymer. For example, each layer may further comprise at least one excipient selected from the group consisting of a disintegrant, a lubricant, a water-soluble additive, a rapid-acting excipient, a filter, and a binder.

A disintegrant acts to absorb water to cause the formulation to rapidly break down into smaller fragments, facilitating dissolution. Examples of the disintegrant useful in the tablet of the present invention include: croscamellose sodium; sodium starch glycolate; pregelatinized starch [Starch 1500 or Prejel]; microcrystalline cellulose; Crospovidone (cross-linked povidone); polyvinylpyrrolidone (PVP, Povidone); hydroxypropylcellulose, low substituted; alginic acid; carboxymethylcellulose, calcium salts and sodium salts; silica, fumed silica, collidal silica; guar gum; magnesium alumimum silicate; methylcellulose, powdered cellulose; starch; sodium alginate; and a combination thereof.

Preferably, the disintegrant may be croscamellose sodium, sodium starch glycolate, pregelatinized starch, microcrystalline cellulose, Crospovidone, and/or polyvinylpyrrolidone. More preferably, Crospovidone, sodium starch glycolate or microcrystalline cellulose may be used as the disintegrant. A combination of two or more of the disintegrants is most preferable. The disintegrant may be added to an oral solid formulation in a pharmaceutically acceptable manner. A secondary disintegrant may be employed to further facilitate rapid release.

The disintegrant may be contained in an amount of 10∼60 % by weight, based on the total weight of the immediate-release layer or the sustained-release layer.

Also, the present invention may employ a lubricant to improve the formability of the sustained-release tablet of levodropropizine as defined in claim 1. Examples of the lubricant include, but are not limited to, magnesium stearate, silica (SiO₂), colloidal silica (Cabo-SIL), and talc. The lubricant may be contained in an amount of 0.25 ∼ 2 % by weight based on the total weight of the immediate-release layer or the sustained-release layer.

The sustained-release tablet of levodropropizine as defined in claim 1 may further comprise a pharmaceutically acceptable water-soluble additive, as exemplified by lactose, sugar, mannitol, lactose, sorbitol, etc.

The water-soluble additive may be contained in an amount of 10∼60 % by weight based on the total weight of the immediate-release layer or the sustained-release layer.

As needed, the sustained-release tablet of levodropropizine as defined in claim 1 may further comprise a preservative and/or a stabilizer.

The sustained-release tablet of levodropropizine according to the present invention is prepared by combining an immediate-release layer and a sustained-release layer into a pellet-type tablet or a multilayered tablet.

Herein disclosed is further a method for preparing a sustained-release tablet of levodropropizine, comprising:
a) blending levodropropizine, a release-controlling polymer, and an additive into sustained-release granules in a wet or dry granulation manner; and
b) postblending the sustained-release granules and a mixture of levodropropizine with a lubricant, and tabletting the same.

In the wet granulation step a), a solvent is added to a powdered blend of levodropropizine, a release-controlling polymer and an additive. The solvent may be selected from the group consisting of: water, ethanol, isopropyl alcohol, glycerin, propylene glycol, polyethylene glycol, and a combination thereof, with water being preferred.

In the tabletting step b), a complex tablet press machine, a multilayer tablet press machine or a double-layer tablet press machine may be used.

As a representative example of the present invention, a bilayer tablet of levodropropizine consisting of an immediate-release layer and a sustained-release layer may be prepared by pressing the granules into a sustained-release layer, followed by applying an immediate-release layer to the sustained-release layer and pressing the immediate-release layer and the sustained-release layer into a tablet. It should be understood that the immediate-release layer need not be pressed after the sustained-release layer is pressed. It is possible to press the immediate-release layer in advance of the formation of the sustained-release layer. Also, granules of the immediate-release layer and the sustained-release layer may be filled in the order stated herein or in an inverse order, followed by a single round of pressing.

In one embodiment, the sustained-release tablet of levodropropizine according to the present invention may be in the form of a bilayer tablet consisting of an immediate-release layer and a sustained-release layer,
or a multilayer tablet comprising a sustained-release core surrounded by an immediate-release layer.

A better understanding of the present invention may be obtained through the following examples which are set forth to illustrate, but are not to be construed as limiting the present invention.

### Mode for Invention

### EXAMPLES

### 1. Test for Determining Amounts of Raw Pharmaceuticals

### 1) Examination on the interaction and compatibility of levodropropizine with excipient

### <Test method>

After being mixed with 90 mg of levodropropizine, the excipients shown in Table 1 were quantitatively analyzed for early content, and then examined for interaction and compatibility with levodropropizine by an acceleration test (temp. 45°C, humidity 75%, duration 1 month).

### <Criterion>

### 95∼105%

**TABLE 1**

| Additive | Early Content (%) | | | Content after Acceleration Test (%) | | |
|---|---|---|---|---|---|---|
| | Criterion | Result | Decision | Criterion | Result | Decision |
| microcrystalline cellulose | 95∼105 | 100.5 | Pass | 95∼105 | 100.3 | Pass |
| Lactose monohydrate | 95∼105 | 100.1 | Pass | 95∼105 | 99.8 | Pass |
| Hydroxypropyl cellulose | 95∼105 | 99.3 | Pass | 95∼105 | 99.2 | Pass |
| hydromellose phthalate | 95∼105 | 99.9 | Pass | 95∼105 | 99.2 | Pass |
| Carboxymethylcellulose sodium | 95∼105 | 99.9 | Pass | 95∼105 | 100 | Pass |
| Carboxymethylcellulose calcium | 95∼105 | 100.2 | Pass | 95∼105 | 99.8 | Pass |
| Copovidone | 95∼105 | 99.3 | Pass | 95∼105 | 99.3 | Pass |
| Aerosil | 95∼105 | 99.5 | Pass | 95∼105 | 99.4 | Pass |
| Poloxamer | 95∼105 | 99.4 | Pass | 95∼105 | 99.4 | Pass |
| *PVP K-30 | 95∼105 | 99.3 | Pass | 95∼105 | 100 | Pass |
| *PVP K-90 | 95∼105 | 99.8 | Pass | 95∼105 | 99.8 | Pass |
| *HPMC2208 (100,000cp) | 95∼105 | 100.3 | Pass | 95∼105 | 99.4 | Pass |
| *HPMC2910 (4000cp) | 95∼105 | 99.7 | Pass | 95∼105 | 99.3 | Pass |
| Crospovidone | 95∼105 | 99.7 | Pass | 95∼105 | 99.2 | Pass |
| Magnesium stearate | 95∼105 | 99.9 | Pass | 95∼105 | 99.3 | Pass |
| Croscamellose sodium | 95∼105 | 99.8 | Pass | 95∼105 | 99.7 | Pass |
| Sodium starch (pregelatinized) | 95∼105 | 99.4 | Pass | 95∼105 | 100 | Pass |
| Hydroxypropyl cellulose, low-substituted | 95∼105 | 99.6 | Pass | 95∼105 | 99.5 | Pass |
| Mannitol | 95∼105 | 99.5 | Pass | 95∼105 | 99.6 | Pass |
| Sodium starch glycolate | 95∼105 | 100.4 | Pass | 95∼105 | 99.8 | Pass |
| Sucrose | 95∼105 | 99.5 | Pass | 95∼105 | 99.5 | Pass |
| Polyvinyl alcohol | 95∼105 | 99.5 | Pass | 95∼105 | 100 | Pass |
| Hydroxyethyl cellulose | 95∼105 | 99.6 | Pass | 95∼105 | 100.3 | Pass |
| Sodium alginate | 95∼105 | 99.5 | Pass | 95∼105 | 99.8 | Pass |

| | | | | | | |
|---|---|---|---|---|---|---|
| *PVP: polyvinylpyrrolidone *HPMC: hydroxypropylmethylcellulose | | | | | | |

### 2) Establishment of immediate-release formulation

### <Test method>

Tablets manufactured by pressing granules in which levodropropizine was blended with excipients as shown in Table 2 were tested for fluidity, hardness, friability, disintegration and dissolution. Based on the test results, suitable compositions for the immediate-release layer were established.

### <Criteria>

Friability: 40 tablets, 100 rotations, 1 % or less
Fluidity: angle of repose, 40°C or less
Hardness: 4∼5kg/cm²
Disintegration: examined with naked eye (disintegration within 5 min), six test specimens for each
Dissolution: 30min-80% or higher

**TABLE 2**

| Immediate formulation | | Friability | Hardness | Fluidity | Disintegration | Dissolution |
|---|---|---|---|---|---|---|
| 1a | levodropropizine (45mg) | Failed | Pass | - | - | - |
| | Flowlac (50mg) | | | | | |
| | magnesium stearate (1.9mg) | | | | | |
| 2a | levodropropizine (45mg) | Failed | Pass | - | - | - |
| | Flowlac (60mg) magnesium | | | | | |
| | stearate (1.9mg) | | | | | |
| 3a | levodropropizine (45mg) | Failed | Pass | - | - | - |
| | Flowlac (80mg) | | | | | |
| | magnesium stearate (1.9mg) | | | | | |
| 5a | levodropropizine (45mg) | Failed | Pass | - | - | - |
| | D-mannitol (60mg) | | | | | |
| | magnesium stearate (1.9mg) | | | | | |
| 6a | levodropropizine (45mg) | Failed | Pass | - | - | - |
| | D-mannitol (80mg) | | | | | |
| | magnesium stearate (1.9mg) | | | | | |
| 7a | levodropropizine (45mg) | Pass | Pass | Failed | - | - |
| | Flowlac (67mg) | | | | | |
| | Corn starch (20mg) | | | | | |
| | magnesium stearate (1.9mg) | | | | | |
| 8a | levodropropizine (45mg) | Pass | Pass | Failed | - | - |
| | Flowlac (67mg) | | | | | |
| | Corn starch (40mg) | | | | | |
| | magnesium stearate (1.9mg) | | | | | |
| 9a | levodropropizine (45mg) | Pass | Pass | Failed | - | - |
| | Flowlac (67mg) | | | | | |
| | Corn starch (60mg) | | | | | |
| | magnesium stearate (1.9mg) | | | | | |
| 10a | levodropropizine (45mg) | Pass | Pass | Failed | - | - |
| | Flowlac (67mg) | | | | | |
| | *MCC pH102 (20mg) | | | | | |
| | magnesium stearate (1.9mg) | | | | | |
| 11a | levodropropizine (45mg) | Pass | Pass | Failed | - | - |
| | Flowlac (67mg) | | | | | |
| | *MCC pH102 (40mg) | | | | | |
| | magnesium stearate (1.9mg) | | | | | |
| 12a | levodropropizine (45mg) | Pass | Pass | Failed | - | - |
| | Flowlac (67mg) | | | | | |
| | MCC pH102 (60mg) | | | | | |
| | magnesium stearate (1.9mg) | | | | | |
| 13a | levodropropizine (45mg) | Pass | Pass | Pass | Pass | Pass |
| | Flowlac (67mg) | | | | | |
| | MCC pH102 (67mg) | | | | | |
| | sodium starch glycolate (5mg) | | | | | |
| | magnesium stearate (1.9mg) | | | | | |
| 14a | levodropropizine (45mg) | Pass | Pass | Pass | Pass | Pass |
| | Flowlac (67mg) | | | | | |
| | MCC pH102 (67mg) | | | | | |
| | sodium starch glycolate (10mg) | | | | | |
| | magnesium stearate (1.9mg) | | | | | |
| 15a | levodropropizine (45mg) | Pass | Pass | Pass | Pass | Failed |
| | Flowlac (67mg) | | | | | |
| | MCC pH102 (67mg) | | | | | |
| | Sodium starch glycolate (20mg) | | | | | |
| | magnesium stearate (1.9mg) | | | | | |
| 16a | levodropropizine (45mg) | Pass | Pass | Pass | Pass | Failed |
| | Flowlac (67mg) | | | | | |
| | MCC pH102 (67mg) | | | | | |
| | CL-PVP (5mg) | | | | | |
| | magnesium stearate (1.9mg) | | | | | |
| 17a | levodropropizine (45mg) | Pass | Pass | Pass | Pass | Failed |
| | Flowlac (67mg) | | | | | |
| | MCC pH102 (67mg) | | | | | |
| | CL-PVP (10mg) | | | | | |
| | magnesium stearate (1.9mg) | | | | | |
| 18a | levodropropizine (45mg) | Pass | Pass | Pass | Pass | Failed |
| | Flowlac (67mg) | | | | | |
| | MCC pH102 (67mg) | | | | | |
| | CL-PVP (20mg) | | | | | |
| | magnesium stearate (1.9mg) | | | | | |
| 19a | levodropropizine (45mg) | Pass | Pass | Pass | Pass | Pass |
| | Flowlac (67mg) | | | | | |
| | MCC pH102 (67mg) | | | | | |
| | croscamellose sodium (5mg) | | | | | |
| | magnesium stearate (1.9mg) | | | | | |
| 20a | levodropropizine (45mg) | Pass | Pass | Pass | Pass | Failed |
| | Flowlac (67mg) | | | | | |
| | MCC pH102 (67mg) | | | | | |
| | Croscarmellose sodium (10mg) | | | | | |
| | magnesium stearate (1.9mg) | | | | | |
| 21a | levodropropizine (45mg) | Pass | Pass | Pass | Pass | Failed |
| | Flowlac (67mg) | | | | | |
| | MCC pH102 (67mg) | | | | | |
| | Croscarmellose sodium (20mg) | | | | | |
| | magnesium stearate (1.9mg) | | | | | |
| 22a | levodropropizine (45mg) | Pass | Pass | Pass | Failed | Failed |
| | Flowlac (67mg) | | | | | |
| | MCC pH102 (67mg) | | | | | |
| | Starch 1500 (5mg) | | | | | |
| | magnesium stearate (1.9mg) | | | | | |
| 23a | levodropropizine (45mg) | Pass | Pass | Pass | Pass | Failed |
| | Flowlac (67mg) | | | | | |
| | MCC pH102 (67mg) | | | | | |
| | Starch 1500 (10mg) | | | | | |
| | magnesium stearate (1.9mg) | | | | | |
| 24a | levodropropizine (45mg) | Pass | Pass | Pass | Pass | Failed |
| | Flowlac (67mg) | | | | | |
| | MCC pH102 (67mg) | | | | | |
| | Starch 1500 (20mg) | | | | | |
| | magnesium stearate (1.9mg) | | | | | |

| | | | | | | |
|---|---|---|---|---|---|---|
| *MCC: microcrystalline cellulose | | | | | | |

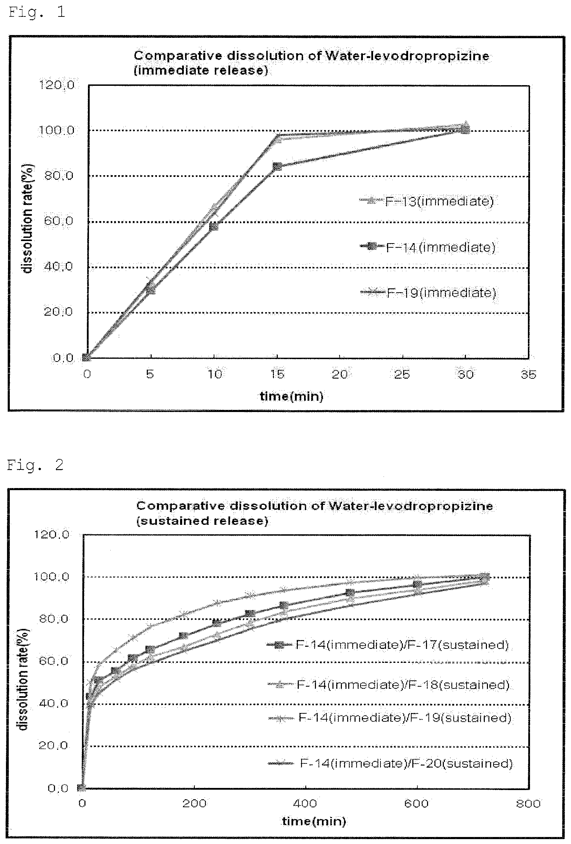

Dissolution profiles of formulations 13a(F-13 (immediate)), 14a(F-14 (immediate)) and 19a(F-19 (immediate)) are depicted in FIG. 1.

In order to improve the pharmaceutical efficacy, it is important to control the dissolution behavior of the immediate-release moiety. A high dissolution rate of the immediate-release moiety makes it difficult for the drug to sufficiently exert pharmaceutical activity because the active ingredient has a half life of as short as 1 hr. Hence, Formulation 14a with a relatively low dissolution rate between 5 min and 15 min after administration was determined as the most suitable immediate-release formulation, based on the data of the test.

In preparing the immediate-release layer of the sustained-release tablet of levodropropizine according to the present invention, it was therefore determined to employ in combination: Flowlac, a product of Meggle, to improve fluidity; microcrystalline cellulose PH102 as a filler; magnesium stearate to prevent sticking; and sodium starch glycolate to increase an early dissolution rate, in combination.

### 3) Establishment of sustained-release formulation

### <Test method>

Together with the immediate-release layer of Formulation 14a, sustained-release layers prepared from wet granules of the formulations given in Table 3 were pressed into bilayered tablets. These tablets were tested for fluidity, hardness, and dissolution to establish formulations suitable for the sustained-release moiety.

### <Criteria>

Fluidity: angle of repose, 40°C or less
Hardness: 4∼5 kg/cm2
Dissolution: 30 min-40∼60%, 180 min-60∼80%, 720 min-85% or higher

**TABLE 3**

| Formulation | | | Hardness | Fluidity | Dissolution |
|---|---|---|---|---|---|
| | Immediate-Release layer | Sustained-Release layer | | | |
| 1b | levodropropizine (45mg) | levodropropizine (45mg) | Pass | Failed | - |
| | Flowlac (67mg) | Kolidon SR (10mg) | | | |
| | *MCC pH102 (67mg) | magnesium stearate (1.7mg) | | | |
| | sodium starch glycolate (10mg) | | | | |
| | magnesium stearate (1.9mg) | | | | |
| 2b | levodropropizine (45mg) | levodropropizine (45mg) | Pass | Failed | - |
| | Flowlac (67mg) | Kolidon SR (30mg) | | | |
| | MCC pH102 (67mg) | magnesium stearate (1.7mg) | | | |
| | sodium starch glycolate (10mg) | | | | |
| | magnesium stearate (1.9mg) | | | | |
| 3b | levodropropizine (45mg) | levodropropizine (45mg) | Pass | Failed | - |
| | Flowlac (67mg) | Kolidon SR (50mg) | | | |
| | MCC pH102 (67mg) | magnesium stearate (1.7mg) | | | |
| | sodium starch glycolate (10mg) | | | | |
| | magnesium stearate (1.9mg) | | | | |
| 4b | levodropropizine (45mg) | levodropropizine (45mg) | Pass | Failed | - |
| | Flowlac (67mg) | HPMC2208 (100,000cps) (10mg) | | | |
| | MCC pH102 (67mg) | magnesium stearate (1.7mg) | | | |
| | sodium starch glycolate (10mg) | | | | |
| | magnesium stearate (1. 9mg) | | | | |
| 5b | levodropropizine (45mg) | levodropropizine (45mg) | Pass | Failed | - |
| | Flowlac (67mg) | HPMC2208 (100,000cps) (30mg) | | | |
| | MCC pH102 (67mg) | magnesium stearate (1.7mg) | | | |
| | sodium starch glycolate (10mg) | | | | |
| | magnesium stearate (1.9mg) | | | | |
| 6b | levodropropizine (45mg) | levodropropizine (45mg) | Pass | Failed | - |
| | Flowlac (67mg) | HPMC2208 (100,000cps) (50mg) | | | |
| | MCC pH102 (67mg) | magnesium stearate (1.7mg) | | | |
| | sodium starch glycolate (10mg) | | | | |
| | magnesium stearate (1.9mg) | | | | |
| 7b | | levodropropizine (45mg) | Pass | Pass | Failed |
| | levodropropizine (45mg) | Lactose (5mg) | | | |
| | Flowlac (67mg) | HPMC2208 (100,000cps) (50mg) | | | |
| | MCC pH102 (67mg) | magnesium stearate (1.7mg) | | | |
| | sodium starch glycolate (10mg) | | | | |
| | magnesium stearate (1.9mg) | | | | |
| 8b | levodropropizine (45mg) | levodropropizine (45mg) | Pass | Pass | Failed |
| | Flowlac (67mg) | Lactose (10mg) | | | |
| | MCC pH102 (67mg) | HPMC2208 (100,000cps) (50mg) | | | |
| | sodium starch glycolate (10mg) | magnesium stearate (1.7mg) | | | |
| | magnesium stearate (1.9mg) | | | | |
| 9b | levodropropizine (45mg) | levodropropizine (45mg) | Pass | Pass | Failed |
| | Flowlac (67mg) | | | | |
| | MCC pH102 (67mg) | Lactose (15mg) | | | |
| | sodium starch glycolate (10mg) | HPMC2208 (100,000cps) (50mg) | | | |
| | magnesium stearate (1.9mg) | magnesium stearate (1.7mg) | | | |
| 10b | levodropropizine (45mg) | levodropropizine (45mg) | Pass | Pass | Failed |
| | Flowlac (67mg) | | | | |
| | MCC pH102 (67mg) | MCC pH 101 (5mg) | | | |
| | sodium starch glycolate (10mg) | HPMC2208 (100,000cps) (50mg) | | | |
| | magnesium stearate (1.9mg) | magnesium stearate (1.7mg) | | | |
| 11b | levodropropizine (45mg) | levodropropizine (45mg) | Pass | Pass | Failed |
| | Flowlac (67mg) | | | | |
| | MCC pH102 (67mg) | MCC pH 101 (10mg) | | | |
| | sodium starch glycolate (10mg) | HPMC2208 (100,000cps) (50mg) | | | |
| | magnesium stearate (1.9mg) | magnesium stearate (1.7mg) | | | |
| 12b | levodropropizine (45mg) | levodropropizine (45mg) | Pass | Pass | Failed |
| | Flowlac (67mg) | | | | |
| | MCC pH102 (67mg) | MCC pH 101 (15mg) | | | |
| | sodium starch glycolate (10mg) | HPMC2208 (100,000cps) (50mg) | | | |
| | magnesium stearate (1.9mg) | magnesium stearate (1.7mg) | | | |
| 13b | levodropropizine (45mg) | levodropropizine (45mg) | Pass | Pass | Failed |
| | Flowlac (67mg) | MCC pH 101 (7mg) | | | |
| | MCC pH102 (67mg) | PVP K-30 (3mg) | | | |
| | sodium starch glycolate (10mg) | HPMC2208 (100,000cps) (50mg) | | | |
| | magnesium stearate (1.9mg) | magnesium stearate (1.7mg) | | | |
| 14b | levodropropizine (45mg) | levodropropizine (45mg) | Pass | Pass | Failed |
| | Flowlac (67mg) | MCC pH 101 (7mg) | | | |
| | MCC pH102 (67mg) | PVP K-30 (6mg) | | | |
| | sodium starch glycolate (10mg) | HPMC2208 (100,000cps) (50mg) | | | |
| | magnesium stearate (1.9mg) | magnesium stearate (1.7mg) | | | |
| 15b | levodropropizine (45mg) | levodropropizine (45mg) | Pass | Pass | Failed |
| | Flowlac (67mg) | MCC pH 101 (7mg) | | | |
| | MCC pH102 (67mg) | PVP K-30 (9mg) | | | |
| | sodium starch glycolate (10mg) | HPMC2208 (100,000cps) (50mg) | | | |
| | magnesium stearate (1.9mg) | magnesium stearate (1.7mg) | | | |
| 16b | levodropropizine (45mg) | levodropropizine (45mg) | Pass | Pass | Failed |
| | Flowlac (67mg) | MCC pH 101 (7mg) | | | |
| | MCC pH102 (67mg) | PVP K-30 (9mg) | | | |
| | sodium starch glycolate (10mg) | HPMC2208 (100,000cps) (60mg) | | | |
| | magnesium stearate (1.9mg) | magnesium stearate (1.7mg) | | | |
| 17b | levodropropizine (45mg) | levodropropizine (45mg) | Pass | Pass | Pass |
| | Flowlac (67mg) | | | | |
| | MCC pH102 (67mg) | MCC pH 101 (7mg) | | | |
| | sodium starch glycolate (10mg) | PVP K-30 (9mg) | | | |
| | magnesium stearate (1.9mg) | HPMC2208 (100,000cps) (80mg) | | | |
| | | magnesium stearate (1.7mg) | | | |
| 18b | levodropropizine (45mg) | levodropropizine (45mg) | Pass | Pass | Pass |
| | Flowlac (67mg) | MCC pH 101 (7mg) | | | |
| | MCC pH102 (67mg) | PVP K-30 (9mg) | | | |
| | sodium starch glycolate (10mg) | HPMC2208 (100,000cps) (100mg) | | | |
| | magnesium stearate (1.9mg) | magnesium stearate (1.7mg) | | | |
| 19b | levodropropizine (45mg) | levodropropizine (45mg) | Pass | Pass | Pass |
| | Flowlac (67mg) | MCC pH 101 (7mg) | | | |
| | MCC pH102 (67mg) | PVP K-90 (3mg) | | | |
| | sodium starch glycolate (10mg) | HPMC2208 (100,000cps) (50mg) | | | |
| | magnesium stearate (1.9mg) | magnesium stearate (1.7mg) | | | |
| 20b | levodropropizine (45mg) | levodropropizine (45mg) | Pass | Pass | Pass |
| | Flowlac (67mg) | MCC pH 101 (7mg) | | | |
| | MCC pH102 (67mg) | PVP K-90 (6mg) | | | |
| | sodium starch glycolate (10mg) | HPMC2208 (100,000cps) (50mg) | | | |
| | magnesium stearate (1.9mg) | magnesium stearate (1.7mg) | | | |
| 21b | levodropropizine (45mg) | levodropropizine (45mg) | Pass | Pass | Failed |
| | Flowlac (67mg) | MCC pH 101 (7mg) | | | |
| | MCC pH102 (67mg) | PVP K-90 (9mg) | | | |
| | sodium starch glycolate (10mg) | HPMC2208 (100,000cps) (50mg) | | | |
| | magnesium stearate (1.9mg) | magnesium stearate (1.7mg) | | | |
| 22b | levodropropizine (45mg) Flowlac (67mg) | levodropropizine (45mg) | Pass | Pass | Failed |
| | MCC pH102 (67mg) | MCC pH 101 (7mg) | | | |
| | sodium starch glycolate (10mg) | PVP K-90 (9mg) | | | |
| | | HPMC 2910 (50mg) | | | |
| | magnesium stearate (1.9mg) | magnesium stearate (1.7mg) | | | |
| 23b | levodropropizine (45mg) | levodropropizine (45mg) | Pass | Pass | Failed |
| | Flowlac (67mg) | | | | |
| | MCC pH102 (67mg) | MCC pH 101 (7mg) | | | |
| | sodium starch glycolate (10mg) | PVP K-90 (9mg) | | | |
| | | HPMC 2910 (80mg) | | | |
| | magnesium stearate (1.9mg) | magnesium stearate (1.7mg) | | | |
| 24b | levodropropizine (45mg) | levodropropizine (45mg) | Pass | Pass | Failed |
| | Flowlac (67mg) | | | | |
| | MCC pH102 (67mg) | MCC pH 101 (7mg) | | | |
| | sodium starch glycolate (10mg) | PVP K-90 (9mg) | | | |
| | | HPMC 2910 (100mg) | | | |
| | magnesium stearate (1.9mg) | magnesium stearate (1.7mg) | | | |

| | | | | | |
|---|---|---|---|---|---|
| *MCC: microcrystalline cellulose | | | | | |

Dissolution profiles of Formulations 17b (F-14(immediate)/F-17(sustained)), 18b(F-14(immediate)/F-18(sustained)), 19b (F-14(immediate)/F-19(sustained)) and 20b(F-14(immediate)/F-20(sustained)) are depicted in FIG. 2

In consideration of the fact that it is important to dissolve the effective drug ingredient at a high rate in the early stages of administration and to maintain a constant effective serum level of the drug to 12 hrs after administration, Formulation 19b was determined as the most ideal formulation because it exhibited the highest early dissolution rate and maintained a stable serum level of the drug.

### 4) Establishment of final formulation

### <Test method>

From data of the above tests, Formulations 14a and 19b were determined to be suitable for the immediate-release layer and the sustained-release layer, respectively. Since dissolution rates of Formulation 19b, however, approximated to the upper and lower limits of the criterion, final formulations were modified to have the excipient contents shown in Table 4, below.

**TABLE 4*)**

| | Final Formulation | Ex. 1 | Ex. 2 | Ex. 3 |
|---|---|---|---|---|
| Sustained Release Layer | levodropropizine | 45.0 | 45.0 | 45.0 |
| | *MCC 101 | 7.0 | 6.8 | 6.8 |
| | *HPMC 2208 | 50.0 | 50.0 | 50.0 |
| | *PVP K-90 | 3.0 | 2.7 | 2.7 |
| | *s-Mg | 1.7 | 1.7 | 1.7 |
| | Total (mg) | 106.7 | 106.2 | 106.2 |
| Immediate-Release Layer | levodropropizine | 45.0 | 45.0 | 45.0 |
| | Flowlac | 67.0 | 67.0 | 67.0 |
| | MCC pH102 | 67.0 | 67.0 | 67.0 |
| | Sodium starch glycolate | 10.0 | 10.0 | 7.5 |
| | s-Mg | 1.9 | 1.9 | 1.9 |
| | Total (mg) | 190.9 | 190.9 | 188.4 |
| Overall Weight (mg) | | 297.6 | 297.1 | 294.6 |

| | | | | |
|---|---|---|---|---|
| *MCC: microcrystalline cellulose *HPMC: hydroxypropylmethylcellulose *) Ex. 1 and Ex. 2 are Reference Examples *PVP K-90: polyvinylpyrrolidone K-90 *s-Mg: magnesium stearate | | | | |

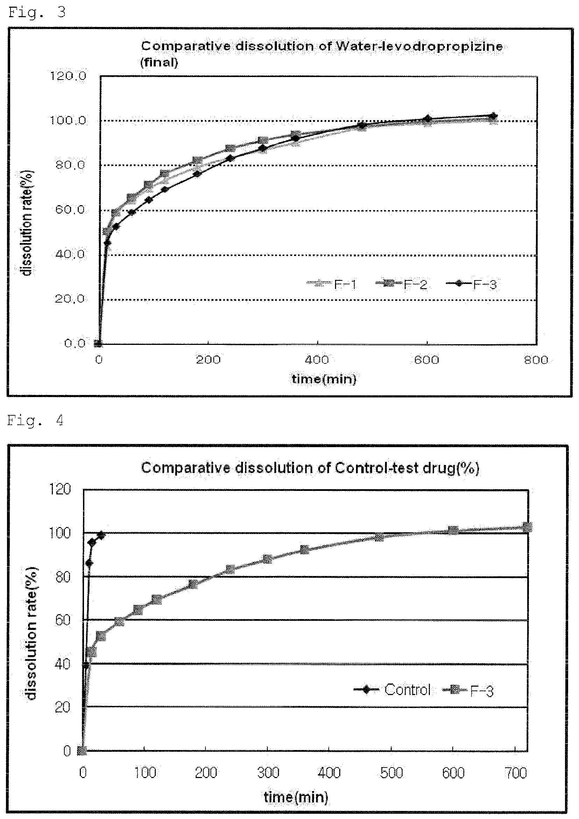

As can be seen in FIG. 3, the formulation of Example 3 given in Table 4 was understood to be the most suitable for the dissolution criterion.

### 2. Non-Clinical Test: Pharmacokinetic Assay in Beagle Dog after Single Oral Dosage

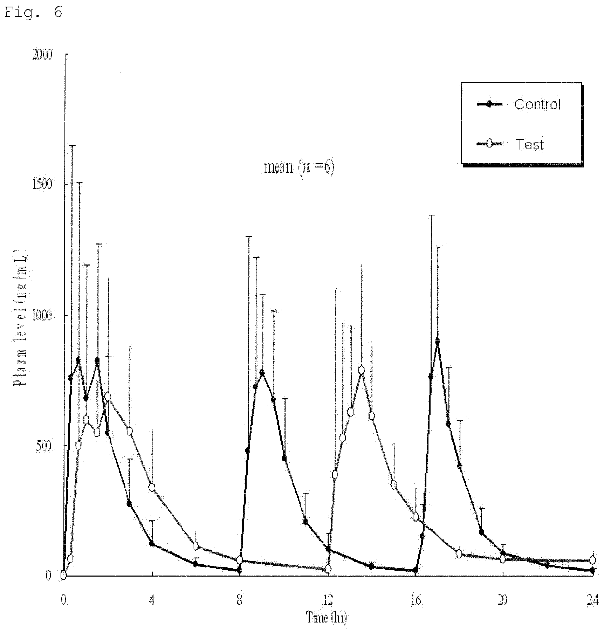

'UI04LDP090CT' of Korea United Pharm. Inc, which has the same composition as the formulation of Example 3 was used as a test formulation while a 'Dropizin' tablet, commercially available from Kolon Pharmaceuticals, Inc., was used as a control. For 24 hrs, three control tablets and two test tablets were orally administered at regular intervals of 8 hrs and 12 hrs, respectively, to beagle dogs. During the test, serum levels of levodropropizine were monitored, and are depicted in FIG. 6.

The control dropizine tablet and the test formulation UI04LDP090CT were observed to have an AUCₜ of 5318.39 and 5722.13 hr*ng/mL, a Cmax of 1205.72 and 1295.53 ng/mL, a Tmax of 1.08 and 0.83 hrs, a t1/2 of 2.43 and 1.89 hrs, respectively, on average.

From the values, percentages of the test material-administered group to the control-administered group were calculated to be 92.9% for AUCₜ and 93.1% for Cmax. That is, the test tablet was not significantly different in AUCₜ and Cmax from the control tablet although the frequency of administration was reduced due to a difference in content therebetween. A higher early serum level of the drug based on the formulation thereof was detected in the control group than in the test group until 1.5 hours post-administration. After that point, however, the test group maintained a higher serum level than did the control group. Therefore, the test formulation was found to exhibit a sustained-release effect in vivo.

### 3. Comparison with Currently Marketed Drug

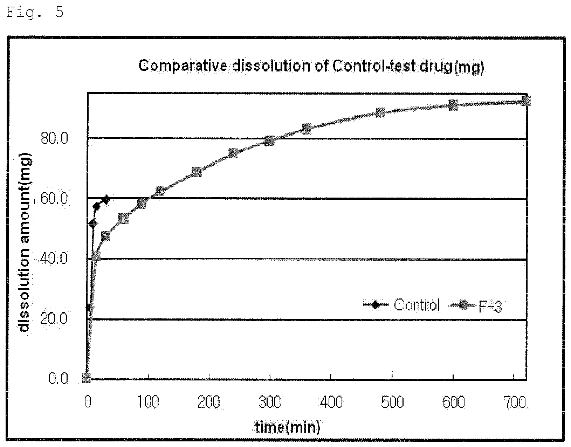

The tablet having the composition of Example 3 in Table 4 and the currently marketed tablet levodropropizine 60 mg were examined for the dissolution behavior of levodropropizine. As can be seen in FIGS. 4 and 5, the currently marketed levodropropizine 60 mg was found to release 60 mg of levedropropizine within 30 min after administration whereas the formulation of Example 3 dissolve 60 mg of levedropropizine within 1 hr, and gradually increased the dissolution to a total of 90 mg until the 12 hr mark, with the maintenance of a constant release rate of levodropropizine.

Having the advantage of reaching a therapeutically effective serum level at an early stage and maintaining the effective serum level for a prolonged duration, the formulation of Example 3 can be administered twice a day based on a dose of 60 mg in contrast to the conventional tablet administered three times a day, which leads to improving patients' convenience and drug compliance.

From data on the early dissolution amount in the in-vitro test, a difference in effect between the test tablet and the currently marketed levodropropizine 60 mg was anticipated, but as measured by the in-vivo test with beagle dogs, the test tablet showed a difference in Tmax of 15 min or less from the levodropropizine 60 mg, and a Cmax corresponding to 90 % of that of the levodropropizine 60 mg.

This is because the half life of levodropropizine itself is short, which makes Tmax values similar. Hence, although its early reaction time is delayed by 60 min compared to the currently marketed levodropropizine 60mg in vitro, the sustained-release tablet of levodropropizine according to the present invention exhibits Cmax and Tmax similar to those of the currently marketed levodropropizine 60mg, in vivo. When administered to humans, the tablet of the present invention is expected to have the same effect and efficacy as the currently marketed levodropropizine 60 mg.

## Claims

1. A sustained-release tablet of levodropropizine, comprising:
an immediate-release layer containing levodropropizine; and
a sustained-release layer containing levodropropizine and a release-controlling polymer,
wherein the sustained-release layer contains 45.0 mg levodropropizine, 6.8 mg microcrystalline cellulose (MCC), 50.0 mg hydroxypropylmethyl cellulose (HPMC), 2.7 mg polyvinylpyrrolidone (PVP), and 1.7 mg magnesium stearate, and
wherein the immediate-release layer contains 45.0 mg levodropropizine, 67.0 mg lactose, 67.0 mg microcrystalline cellulose (MCC), 7.5 mg sodium starch glycolate, and 1.9 mg magnesium stearate.

## Patentansprüche

1. Eine Tablette mit verzögerter Freisetzung mit Levodropropizin, umfassend:
eine Schicht mit sofortiger Freisetzung, die Levodropropizin enthält; und
eine Schicht mit verzögerter Freisetzung, die Levodropropizin und ein die Freisetzung regulierendes Polymer enthält,
wobei die Schicht mit verzögerter Freisetzung 45,0 mg Levodropropizin, 6,8 mg mikrokristalline Cellulose (MCC), 50,0 mg Hydroxypropylmethylcellulose (HPMC), 2,7 mg Polyvinylpyrrolidon (PVP) und 1,7 mg Magnesiumstearat enthält, und
wobei die Schicht mit sofortiger Freisetzung 45,0 mg Levodropropizin, 67,0 mg Laktose, 67,0 mg mikrokristalline Cellulose (MCC), 7,5 mg Natriumstärkeglycolat und 1,9 mg Magnesiumstearat enthält.

## Revendications

1. Comprimé à libération prolongée de lévodropropizine, comprenant :
une couche à libération immédiate contenant de la lévodropropizine et
une couche à libération prolongée contenant de la lévodropropizine et un polymère régulant la libération,
où la couche à libération prolongée contient 45,0 mg de lévodropropizine, 6,8 mg de cellulose microcristalline (CMC), 50,0 mg d'hydroxypropyl-méthylcellulose (HPMC), 2,7 mg de polyvinylpyrrolidone (PVP) et 1,7 mg de stéarate de magnésium, et
où la couche à libération immédiate contient 45,0 mg de lévodropropizine, 67,0 mg de lactose, 67,0 mg de cellulose microcristalline (CMC), 7,5 mg de glycolate d'amidon sodique et 1,9 mg de stéarate de magnésium.
